# EUROPÄISCHE PATENTSCHRIFT

(11) **EP 3 031 813 B1**
(45) Veröffentlichungstag und Bekanntmachung des Hinweises auf die Patenterteilung: **23.11.2016**
(21) Anmeldenummer: 15178263.8
(22) Anmeldetag: 04.12.2014
(51) Int. Cl.: C07F 9/6574, C07C 45/50, B01J 31/28

(54) **MONOPHOSPHITE DIE EIN MENTHOL AUFWEISEN**
MONOPHOSPHITES WITH A MENTHOL
MONOPHOSPHITES PRESENTANT DU MENTHOL

(43) Veröffentlichungstag der Anmeldung: 15.06.2016
(62) Teilanmeldung aus: 14196188.8
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Börner, Armin, 18059 Rostock (DE); Selent, Detlef, 18059 Rostock (DE)

(56) Entgegenhaltungen:
- WO-A2-2004/076464
- T. BARTIK ET AL: "Control of asymmetric induction in the organometallic synthesis of 3-methyl-(E)-4-hexen-2-one", CHIRALITY, Bd. 3, Nr. 4, 1991, Seiten 324-330, XP055199536, ISSN: 0899-0042, DOI: 10.1002/chir.530030418
- CHEN W ET AL: "Enantioselective hydrogenation with inexpensive, easily available monodentate phosphite ligands", TETRAHEDRON LETTERS, PERGAMON, GB, Bd. 42, Nr. 15, 9. April 2001 (2001-04-09) , Seiten 2897-2899, XP004232343, ISSN: 0040-4039, DOI: 10.1016/S0040-4039(01)00319-7

## Beschreibung

Die Erfindung betrifft Monophosphite, die ein Menthol aufweisen. Des Weiteren deren Verwendung als Liganden in der Hydroformylierung.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P(III). Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Synthese von symmetrischen Monophosphiten, die ein Menthol enthalten wurde bereits in "Rh-Catalyzed Asymmetrie Hydrogenation of Prochiral Olefins with a Dynamic Library of Chiral TROPOS Phosphorus Ligands" von Chiara Monti, Cesare Gennari, Umberto Piarulli, Johannes G. de Vries, Andre H. M. de Vries und Laurent Lefort in Chem. Eur. J. 2005, 11, 6701 - 6717 beschrieben. Hierbei wurden lediglich symmetrische Verbindungen hergestellt, die ein symmetrisches Biphenol oder Binaphthol enthalten. Diese Verbindungen wurden dann in der asymmetrischen Hydrierung getestet.

In WO 2004/076464 A2 werden optisch aktive Phosphite und Phosphoramidite offenbart, mit welchen asymmetrische Reaktionen katalysiert werden können.

T. Bartik et al. "Control of asymmetric induction in the organometallic synthesis of 3-methyl-(E)-4-hexen-2-one", Chirality, Bd. 3, Nr. 4, 1. Januar 1991, Seiten 324-330, offenbart ebenfalls Liganden für die asymmetrische Katalyse.

Chen W. et al. "Enantioselective hydrogenation with inexpensive, easily available monodentate phosphite ligands", Tetrahedron Letters, Pergamon, GB, Bd. 42, Nr. 15, 9. April 2001, Seiten 2897-2899, offenbart reine Diastereomere von Binaphthyl-Phosphit-Liganden.

Der Erfindung lag die Aufgabe zugrunde, Monophosphite bereitzustellen, welche in der Hydroformylierung als Liganden eingesetzt werden können. Insbesondere bestand die Aufgabe darin, neue Liganden bereitzustellen, deren Einsatz zu einer guten Ausbeute führt. Die gute Ausbeute sollte bei zumindest einem Olefin realisiert werden.

Die Aufgabe wird gelöst durch eine Verbindung nach Anspruch 1.

Verbindung, welche die allgemeinen Strukturen (II) aufweist: 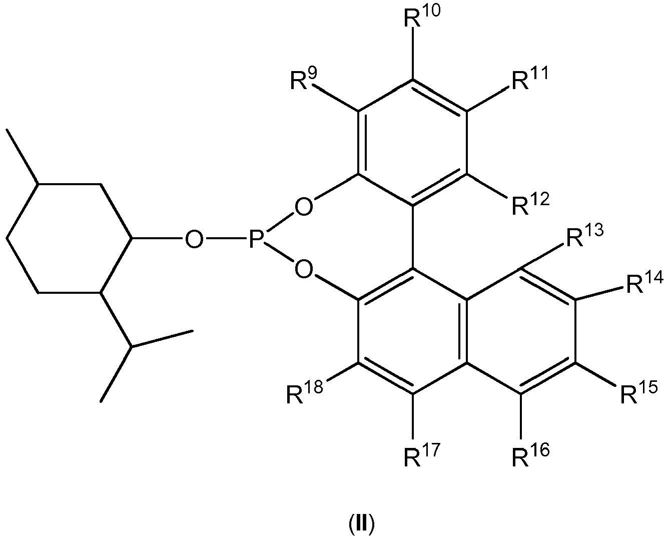 wobei
R⁹, R¹⁰, R¹¹, R¹², R¹³, R¹⁴, R¹⁵, R¹⁶, R¹⁷, R¹⁸ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
   substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H;-SO₃Na, -NO₂, -CN, -NH₂, -N[(C₁-C₁₂)-Alkyl]₂.
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.
(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I),-COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂₎-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.
Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-, Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter - H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I),-COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂), Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte-(C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl, -O-(C₆-C₂₀)-Aryl.

In einer Ausführungsform sind R⁹, R¹⁰, R¹¹, R¹², R¹³, R¹⁴, R¹⁵, R¹⁶, R¹⁷, R¹⁸ ausgewählt aus: -H, -(C₁-C₁₂)-Alkyl, -O-(C₁-C₁₂)-Alkyl.

In einer Ausführungsform weist die Verbindung die Struktur (**1**) auf:

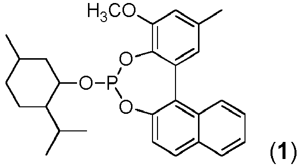

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

In einer bevorzugten Variante des Verfahrens ist das Metallatom Rh.

Es kann hierbei auch ein Überschuss an Liganden verwendet werden und nicht zwangsläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 200 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2-oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Im Folgenden wird die Erfindung anhand von Ausführungsbeispielen näher erläutert.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Biphenole

Die Synthese der Biphenole erfolgt analog zu DE102013203865 und DE102013203867.

### Synthese der Chlorophosphite

Die Synthese der Mono- und Dichlorophosphite wie Dichlor((-)-menthyloxy)phosphin oder 2-Chloro-4,4,5,5-tetraphenyl-1,3,2-dioxaphospholan ist dem Fachmann bekannt und erfolgt nach bekannter Weise. Chlorophosphite lassen sich durch Zusatz von Phosphortrichlorit in Gegenwart einer Base herstellen. Für nähere Informationen siehe auch "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### Synthese der Monophosphite

### 6-((2-Isopropyl-5-methylcyclohexyl)oxy)-4-methoxy-2-methylbenzo[d]naphtho[1,2-f][1,3,2]dioxaphosphepin.

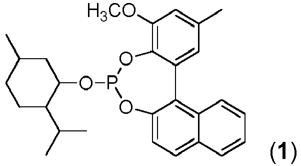

Eine gerührte Suspension von 1-(2-Hydroxy-3-methoxy-5-methylphenyl)naphthalen-2-ol (0,654 g; 2,33 mmol) in Toluol (16 ml) wurde mit Triethylamin (2,17 g; 21,41 mmol) versetzt und die erhaltene Mischung tropfenweise bei 0 °C zu einer Lösung von Dichlor((-)-menthyloxy)phosphin (0,597 g; 2,33 mmol) in Toluol (15 ml) gegeben. Die Reaktionsmischung wurde über Nacht bei Raumtemperatur und danach für 2 h bei 70 °C gerührt. Die Mischung wurde filtriert und das Filtrat im Vakuum bis zur Trockne eingedampft. Ausbeute: 0,859 g (1,849 mmol; 79 %). Elementaranalyse (berechnet für C₂₈H₃₃O₄P = 464,51 g/ mol) C 72,38 (72,39); H 7,43 (7,16) %.
³¹P-NMR (CD₂Cl₂): 152,1; 154,7; 155,1; 158,5 ppm.
¹H-NMR (CD₂Cl₂): 0,85-1,08 (m, 10 H); 1,08-1,64 (m, 4 H); 1,65-1,81 (m, 2 H); 2,09-2,38 (m, 2 H); 2,47 (s, 3 H); 3,93-3,98 (m, 3 H); 4,12-4,32 (m, 1 H); 6,92 (m, 1 H, Hₐᵣₒₘ); 7,04-7,07 (m, 1 H, Hₐᵣₒₘ); 7,26-7,34 (m, 1 H, Hₐᵣₒₘ); 7,52 (m, 2 H, Hₐᵣₒₘ); 7,88-7,96 (m, 2 H, Hₐᵣₒₘ); 8,14-8,20 (m, 1 H, Hₐᵣₒₘ) ppm.
ESI-TOF/HRMS: m/e 487,20091 (M+Na)⁺.

### 9-(tert-Butyl)-6-((2-isopropyl-5-methylcyclohexyl)oxy)-4-methoxy-2-methyldibenzo[d,f][1,3,2]dioxaphosphepin. (Vergleichsligand)

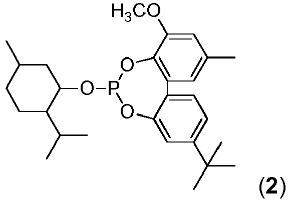

Eine gerührte Suspension von 4'-(*tert*-Butyl)-3-methoxy-5-methyl-[1,1'-biphenyl]-2,2'-diol (0,675 g; 2,36 mmol) in Toluol (16 ml) wurde mit Triethylamin (2,188 g; 21,62 mmol) versetzt und die erhaltene Mischung tropfenweise bei 0 °C zu einer Lösung von Dichlor((-)-menthyloxy)phosphin (0,606 g; 2,36 mmol) in Toluol (15 ml) gegeben. Die Reaktionsmischung wurde über Nacht gerührt und filtriert. Das Filtrat wurde im Vakuum zur Trockne eingeengt. Ausbeute: 0,965 g (2,051 mmol; 87 %). Elementaranalyse (berechnet für C₂₈H₃₉O₄P = 470,56 g/ mol) C 71,49 (71,46); H 8,59 (8,35); P 6,81 (6,58) %.
³¹P-NMR (CD₂Cl₂): 153,2; 153,4 ppm.
¹H-NMR (CD₂Cl₂): (m, 4 H); 0,97-1,02 (m, 6 H); 1,04-1,39 (m, 3 H); 1,41 (m, 9 H); 1,46-1,61 (m, 1 H); 1,73 (m, 2 H); 2,19-2,37 (m, 2 H); 2,43 (s, 3 H); 3,91 (s, 3 H); 4,15-4,28 (m, 1 H); 6,87 (m, 2 H, Hₐᵣₒₘ); 7,20-7,21 (m, 1 H, Hₐᵣₒₘ); 7,33-7,36 (m, 1 H, Hₐᵣₒₘ); 7,43-7,45 (m, 1 H, Hₐᵣₒₘ) ppm. ESI-TOF/HRMS: m/e 493,24844 (M+Na)⁺.

### 2-Isopropyl-6-((2-isopropyl-5-methylcyclohexyl)oxy)-8-methoxy-3,10-dimethyldibenzo[d,f][1,3,2]dioxaphosphepin. (Vergleichsligand)

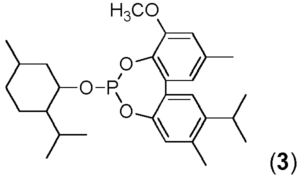

Eine Lösung von 5'-Isopropyl-3-methoxy-4',5-dimethyl-[1,1'-biphenyl]-2,2'-diol (0,664 g; 2,32 mmol) in Toluol (16 ml) wurde mit Triethylamin (2,152 g; 21,27 mmol) versetzt und die erhaltene Mischung tropfenweise bei 0 °C zu einer Lösung von Dichlor((-)-menthyloxy)phosphin (0,596 g; 2,32 mmol) in Toluol (15 ml) gegeben. Die Reaktionsmischung wurde über Nacht gerührt und filtriert. Das Filtrat wurde im Vakuum zur Trockne eingeengt. Ausbeute: 0,944 g (2,006 mmol; 87 %).

Elementaranalyse (berechnet für C₂₈H₃₉O₄P = 470,56 g/ mol) C 71,28 (71,46); H 8,36 (8,35); P 6,72 (6,58) %.
³¹P-NMR (CD₂Cl₂): 152,4; 153,7 ppm.
¹H-NMR (CD₂Cl₂): (m, 4 H); 0,97-1,02 (m, 6 H); 1,06-1,29 (m, 2 H); 1,32 (m, 6 H); 1,35-1,46 (m, 1 H); 1,46-1,60 (m, 1 H); 1,73 (m, 2 H); 2,19-2,37 (m, 2 H); 2,40-2,46 (m, 6 H); 3,21 (s, 1 H); 3,90 (s, 3 H); 4,20 (m, 1 H); 6,84 (s, 1 H, Hₐᵣₒₘ); 6,90 (m, 1 H, Hₐᵣₒₘ); 6,96 (m, 1 H, Hₐᵣₒₘ); 7,35 (m, 1 H, Hₐᵣₒₘ) ppm.
ESI-TOF/HRMS: m/e 493,24846 (M+Na)⁺.

### 6-((2-Isopropyl-5-methylcyclohexyl)oxy)-4-methoxy-2,10-dimethyldibenzo[d,f][1,3,2]dioxaphosphepin. (Vergleichsligand)

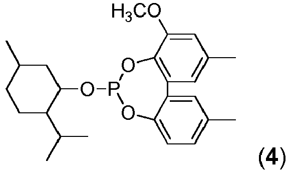

Eine gerührte Suspension von 3-Methoxy-5,5'-dimethyl-[1,1'-biphenyl]-2,2'-diol (0,56 g; 2,3 mmol) in Toluol (16 ml) wurde mit Triethylamin (2,132 g; 21,07 mmol) versetzt und die erhaltene Mischung tropfenweise bei 0 °C zu einer Lösung von Dichlor((-)-menthyloxy)phosphin (0,591 g; 2,3 mmol) in Toluol (15 ml) gegeben. Die Reaktionsmischung wurde über Nacht gerührt und filtriert. Das Filtrat wurde im Vakuum bis zur Trockne eingeengt. Ausbeute: 0,860 g (2,01 mmol; 87 %).

Elementaranalyse (berechnet für C₂₅H₃₃0₄P = 428,48 g/ mol) C 70,26 (70,07); H 7,71 (7,76); P 7,09 (7,23) %.
³¹P-NMR (CD₂Cl₂): 152,9; 153,8 ppm.
¹H-NMR (CD₂Cl₂): (m, 4 H); 0,96-1,01 (m, 6 H); 1,04-1,46 (m, 3 H); 1,46-1,59 (m, 1 H); 1,72 (m, 2 H); 2,17-2,37 (m, 2 H); 2,43 (m, 6 H); 3,90 (s, 3 H); 4,20 (m, 1 H); 6,85 (m, 1 H, Hₐᵣₒₘ); 6,90 (m, 1 H, Hₐᵣₒₘ); 7,06 (m, 1 H, Hₐᵣₒₘ); 7,20 (m, 1 H, Hₐᵣₒₘ); 7,31 (m, 1 H, Hₐᵣₒₘ) ppm.
ESI-TOF/HRMS: m/e 451,20169 (M+Na)⁺.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Als Olefin wurde ein Octengemisch eingesetzt: n-Octene (Oxeno GmbH, Octenisomerengemisch aus 1-Octen: ~3 %; *cis*+*trans*-2-Octen: ~49%; *cis*+*trans*-3-Octen: ~29%; *cis*+*trans*-Octen-4: ~16%; gerüstisomere Octene: ~3 % wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien, Umicore) als Katalysatorvorstufe in Toluol eingefüllt: Für Versuche mit 100 ppm-m Rhodium 10 ml einer 4,31 millimolaren, für 40 bzw. 60 ppm-m die gleiche Menge einer entsprechend verdünnten Lösung. Anschließend wurde die entsprechende Menge der in Toluol gelösten Phosphitverbindung (5 Ligandäquivalente pro Rhodium) zugemischt. Durch Zugabe von weiterem Toluol (Die Gesamtmasse an Toluol wurde bestimmt für die GC-Analyse, s.u.) wurde das Anfangsvolumen der Katalysatorlösung auf 41,0 ml eingestellt. Die jeweils eingebrachte Masse an Toluol wurde bestimmt. Einwaage n-Octene: 10,70 g (95,35 mmol). DerAutoklavwurde bei einem Gesamtgasdruck (Synthesegas: Linde; H₂ (99,999%): CO (99,997%) = 1:1) von a) 42 bar für einen Enddruck von 50 bar bzw. b) 12 bar für den Enddruck von 20 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar bzw. b) 19,5 bar für einen Enddruck von 20 bar erhöht und das Edukt mit einem in der Druckpipette eingestellten Überdruck von ca. 3 bar zugepresst. Die Reaktion wurde bei konstantem Druck von jeweils 50 bzw. 20 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Katalyseergebnisse

Die Ergebnisse der Katalyseversuche sind in Tabelle 1 zusammengefasst.

**Tabelle 1:**

| **Ligand** | **p (bar)** | **T(°C)** | **t (h)** | **[Rh] (ppm)** | **L/Rh** | **Ausb. (%)** |
|---|---|---|---|---|---|---|
| **1*** | 50 | 120 | 4 | 100 | 5 | 96 |
| **2** | 50 | 120 | 4 | 100 | 5 | 90 |
| **3** | 50 | 120 | 4 | 100 | 5 | 95 |
| **4** | 50 | 120 | 4 | 100 | 5 | 92 |

| | | | | | | |
|---|---|---|---|---|---|---|
| * erfindungsgemäße Verbindung Olefin: n-Octene (Oxeno GmbH) Solvens: Toluol Verhältnis Ligand/Rhodium (L/Rh): 5 : 1 | | | | | | |

Wie aus Tabelle 1 ersichtlich ist, konnte mit allen erfindungsgemäßen Verbindungen eine sehr gute Ausbeute erzielt werden.

Anhand der oben beschriebenen Versuche konnte gezeigt werden, dass die gestellte Aufgabe durch die erfindungsgemäßen Verbindungen gelöst wird.

## Patentansprüche

1. Verbindung, welche die allgemeinen Strukturen (II) aufweist: 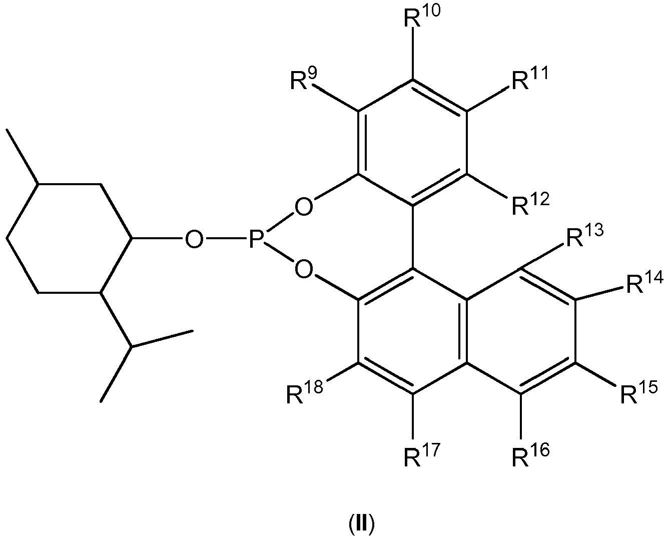 wobei
R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -COO-(C₁-C₁₂)-Alkyl, -CONH-(C₁-C₁₂)-Alkyl, -CO-(C₁-C₁₂)-Alkyl, -CO-(C₆-C₂₀)-Aryl, -COOH, -OH, -SO₃H, -NH₂, -N[(C₁-C₁₂)-Alkyl]₂;
wobei die genannten Alkyl- und Arylgruppen wie folgt substituiert sein können:
substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen; die Substituenten sind unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H;-SO₃Na, -NO₂, -CN, -NH₂, -N[(C₁-C₁₂)-Alkyl]₂.

2. Verbindung nach Anspruch 1,
wobei R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, -CO-(C₁-C₁₂)-Alkyl, -CO-(C₆-C₂₀)-Aryl, -N[(C₁-C₁₂)-Alkyl]₂.

3. Verbindung nach einem der Ansprüche 1 oder 2,
wobei R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen.

4. Verbindung nach einem der Ansprüche 1 bis 3,
wobei R⁹, R¹⁰, R¹¹, R¹², R¹³, R¹⁴, R¹⁵, R¹⁶, R¹⁷, R¹⁸ ausgewählt sind aus:
-H, -(C₁-C₁₂)-Alkyl, -O-(C₁-C₁₂)-Alkyl, -O-(C₆-C₂₀)-Aryl.

5. Verbindung nach einem der Ansprüche 1 bis 4, wobei R⁹, R¹⁰, R¹¹, R¹², R¹³, R¹⁴, R¹⁵, R¹⁶, R¹⁷, R¹⁸ ausgewählt sind aus:
- H, -(C₁-C₁₂)-Alkyl, -O-(C₁-C₁₂)-Alkyl.

6. Verbindung nach einem der Ansprüche 1 bis 5,
welche die Strukture (**1**) aufweist:

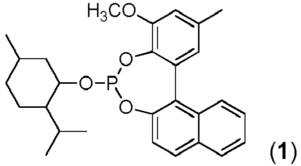

7. Komplex umfassend:
- eine Verbindung nach einem der Ansprüche 1 bis 6,
- ein Metallatom ausgewählt aus: Rh, Ru, Co, Ir.

8. Verwendung einer Verbindung nach einem der Ansprüche 1 bis 6,
zur Katalyse einer Hydroformylierungsreaktion.

9. Verfahren umfassend die Verfahrensschritte:
a) Vorlegen eines Olefins,
b) Zugabe eines Komplexes nach Anspruch 7,
oder einer Verbindung nach einem der Ansprüche 1 bis 6 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having the general structure (II): 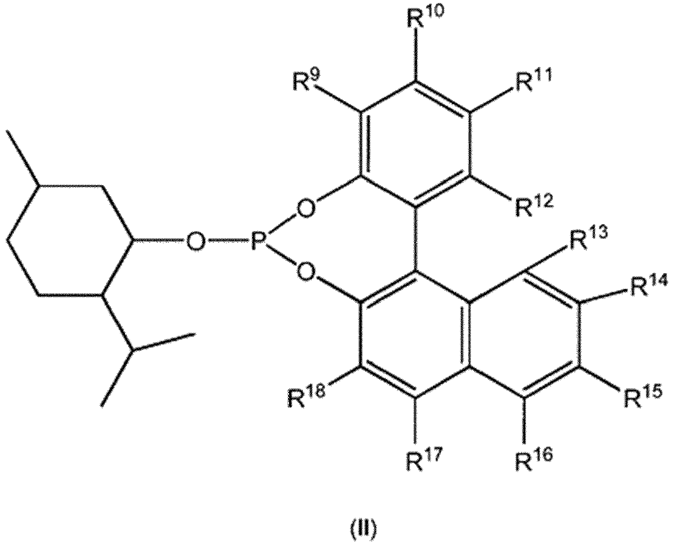 where
R⁹, R¹⁰, R¹¹, R¹², R¹³, R¹⁴, R¹⁵, R¹⁶, R¹⁷, R¹⁸ are selected from:
- H_{,} -(C₁-C₁₂)-alkyl, -O-(C₁-C₁₂)-alkyl, -0- (C₆-C₂₀) -aryl,-(C₆-C₂₀)-aryl, -S-alkyl, -S-aryl, halogen, -COO-(C₁-C₁₂)-alkyl, -CONH-(C₁-C₁₂)-alkyl, -CO-(C₁-C₁₂)-alkyl, -CO-(C₆-C₂₀)-aryl, -COOH, -OH, -SO₃H, -NH₂, -N[(C₁-C₁₂)-alkyl]₂;
wherein the alkyl and aryl groups mentioned may be substituted as follows: substituted -(C₁-C₁₂)-alkyl groups and substituted -(C₁-C₁₂)-alkoxy groups may have one or more substituents depending on their chain length;
the substituents are mutually independently selected from -(C₃-C₁₂)-cycloalkyl, - (C₃-C₁₂)-heterocycloalkyl, - (C₆-C₂₀)-aryl, fluorine, chlorine, cyano, formyl, acyl or alkoxycarbonyl;
substituted -(C₆-C₂₀)-aryl groups and -(C₆-C₂₀)-aryl-(C₆-C₂₀)-aryl groups may have one or more substituents depending on the ring size; these substituents are mutually independently selected from -H, -(C₁-C₁₂)-alkyl, -O-(C₁-C₁₂)-alkyl, -O-(C₆-C₂₀)-aryl, -(C₆-C₂₀)-aryl, halogen, -COO-(C₁-C₁₂)-alkyl, -CONH-(C₁-C₁₂)-alkyl, -(C₆-C₂₀)-aryl-CON[(C₁-C₁₂)-alkyl]₂, -CO-(C₁-C₁₂)-alkyl, -CO-(C₆-C₂₀)-aryl, -COOH, -OH, -SO₃H; - SO₃Na, -NO₂, -CN, -NH₂, -N[(C₁-C₁₂)-alkyl]₂.

2. Compound according to Claim 1,
where R⁹, R¹⁰, R¹¹, R¹², R¹³, R¹⁴, R¹⁵, R¹⁶, R¹⁷, R¹⁸ are selected from:
-H, -(C₁-C₁₂)-alkyl, -O-(C₁-C₁₂)-alkyl, -O-(C₆-C₂₀)-aryl,-(C₆-C₂₀)-aryl, -S-alkyl, -S-aryl, halogen, -CO-(C₁-C₁₂)-alkyl, -CO-(C₆-C₂₀)-aryl, -N[(C₁-C₁₂)-alkyl]₂.

3. Compound according to either of Claims 1 and 2,
where R⁹, R¹⁰, R¹¹, R¹², R¹³, R¹⁴, R¹⁵, R¹⁶, R¹⁷, R¹⁸ are selected from:
-H, -(C₁-C₁₂)-alkyl, -O-(C₁-C₁₂)-alkyl, -O-(C₆-C₂₀)-aryl,-(C₆-C₂₀)-aryl, -S-alkyl, -S-aryl, halogen.

4. Compound according to any of Claims 1 to 3,
where R⁹, R¹⁰, R¹¹, R¹², R¹³, R¹⁴, R¹⁵, R¹⁶, R¹⁷, R¹⁸ are selected from:
-H, -(C₁-C₁₂)-alkyl, -O-(C₁-C₁₂)-alkyl, -0- (C₆-C₂₀) -aryl.

5. Compound according to any of Claims 1 to 4,
where R⁹, R¹⁰, R¹¹, R¹², R¹³, R¹⁴, R¹⁵, R¹⁶, R¹⁷, R¹⁸ are selected from:
-H, -(C₁-C₁₂)-alkyl, -O-(C₁-C₁₂)-alkyl.

6. Compound according to any of Claims 1 to 5 having the structure (1) :

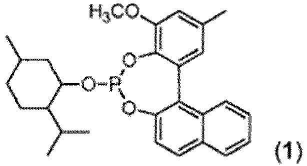

7. Complex comprising:
- a compound according to any of Claims 1 to 6,
- a metal atom selected from: Rh, Ru, Co, Ir.

8. Use of a compound according to any of Claims 1 to 6 for catalysis of a hydroformylation reaction.

9. Method comprising the method steps of:
a) initially charging an olefin,
b) adding a complex according to Claim 7, or a compound according to any of Claims 1 to 6 and a substance having a metal atom selected from: Rh, Ru, Co, Ir,
c) feeding in of H₂ and CO,
d) heating the reaction mixture whereupon the olefin is converted to an aldehyde.

## Revendications

1. Composé qui présente la structure générale (II) : 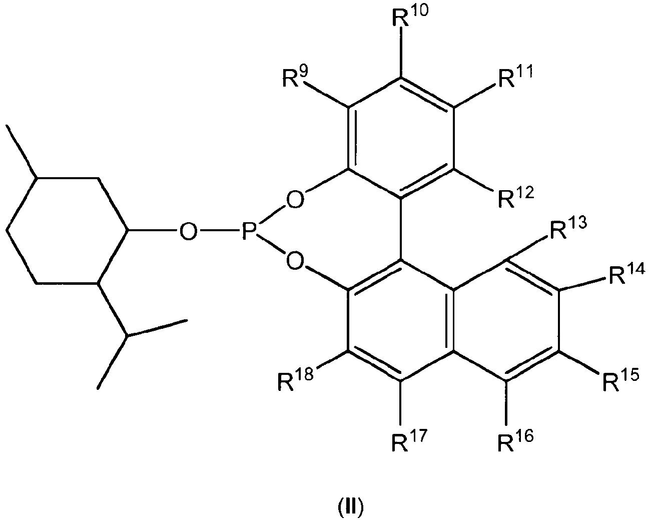 dans laquelle
R⁹, R¹⁰, R¹¹, R¹², R¹³, R¹⁴, R¹⁵, R¹⁶, R¹⁷, R¹⁸ sont choisis parmi :
- H, - (C₁-C₁₂)-alkyle, -O-(C₁-C₁₂)-alkyle, -O-(C₆-C₂₀)-aryle, -(C₆-C₂₀) -aryle, -S-alkyle, -S-aryle, halogène, -COO- (C₁-C₁₂) -alkyle, -CONH- (C₁-C₁₂)-alkyle, -CO- (C₁-C₁₂) -alkyle, -CO- (C₆-C₂₀)-aryle, -COOH, -OH, -SO₃H, -NH₂, -N[(C₁-C₁₂)-alkyle]₂ ;
les groupes alkyle et aryle mentionnés pouvant être substitués comme suit :
les groupes -(C₁-C₁₂)-alkyle substitués et les groupes -(C₁-C₁₂)-alcoxy substitués peuvent présenter, en fonction de leur longueur de chaîne, un ou plusieurs substituants ; les substituants sont choisis, indépendamment les uns des autres, parmi -(C₃-C₁₂)-cycloalkyle, -(C₃-C₁₂)-hétérocycloalkyle, -(C₆-C₂₀)-aryle, fluor, chlore, cyano, formyle, acyle ou alcoxycarbonyle ;
les groupes -(C₆-C₂₀)-aryle substitués et les groupes - (C₆-C₂₀) -aryl- (C₆-C₂₀) -aryle substitués peuvent présenter, en fonction de leur dimension de cycle, un ou plusieurs substituants ; ces substituants sont choisis, indépendamment les uns des autres, parmi -H, - (C₁-C₁₂)-alkyle, -O-(C₁-C₁₂)-alkyle, -O-(C₆-C₂₀)-aryle, -(C₆-C₂₀)-aryle, -halogène, -COO-(C₁-C₁₂)-alkyle, -CONH-(C₁-C₁₂)-alkyle, - (C₆-C₂₀) -aryl-CON[(C₁-C₁₂)-alkyle]₂, -CO-(C₁-C₁₂)-alkyle, -CO-(C₆-C₂₀)-aryle, -COOH, -OH, -SO₃H, -SO₃Na, -NO₂, -CN, -NH₂, -N[(C₁-C₁₂)-alkyle]₂.

2. Composé selon la revendication 1, R⁹, R¹⁰, R¹¹, R¹², R¹⁷, R¹⁴, R¹⁵, R¹⁶, R¹⁷, R¹⁸ étant choisis parmi :
-H, - (C₁-C₁₂) -alkyle, -O-(C₁-C₁₂)-alkyle, -O-(C₆-C₂₀)-aryle, - (C₆-C₂₀) -aryle, -S-alkyle, -S-aryle, halogène, -CO- (C₁-C₁₂) -alkyle, -CO- (C₆-C₂₀) -aryle, -N[(C₁-C₁₂)-alkyle]₂.

3. Composé selon l'une quelconque des revendications 1 ou 2, R⁹, R¹⁰, R¹¹, R¹², R¹⁰, R¹⁴, R¹⁵, R¹⁰, R¹⁷, R¹⁸ étant choisis parmi :
-H, -(C₁-C₁₂) -alkyle, -O-(C₁-C₁₂)-alkyle, -O-(C₆-C₂₀)-aryle, -(C₆-C₂₀)-aryle, -S-alkyle, -S-aryle, halogène.

4. Composé selon l'une quelconque des revendications 1 à 3, R⁹, R¹⁰, R¹¹, R¹², R¹⁰, R¹⁴, R¹⁵, R¹⁰, R¹⁷, R¹⁸ étant choisis parmi :
-H, -(C₁-C₁₂) -alkyle, -O-(C₁-C₁₂)-alkyle, -O-(C₆-C₂₀)-aryle.

5. Composé selon l'une quelconque des revendications 1 à 4, R⁹, R¹⁰, R¹¹, R¹², R¹⁰, R¹⁴, R¹⁵, R¹⁰, R¹⁷, R¹⁸ étant choisis parmi :
-H, - (C₁-C₁₂) -alkyle, -O-(C₁-C₁₂)-alkyle.

6. Composé selon l'une quelconque des revendications 1 à 5, qui présente la structure (1) :

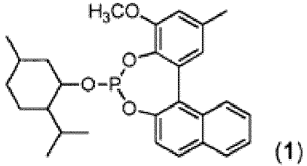

7. Complexe, comprenant :
- un composé selon l'une quelconque des revendications 1 à 6,
- un atome métallique choisi parmi : Rh, Ru, Co, Ir.

8. Utilisation d'un composé selon l'une quelconque des revendications 1 à 6 pour la catalyse d'une réaction d'hydroformylation.

9. Procédé comprenant les étapes de procédé :
a) disposition préalable d'une oléfine,
b) addition d'un complexe selon la revendication 7,
ou d'un composé selon l'une quelconque des revendications 1 à 6 et d'une substance qui présente un atome métallique choisi parmi : Rh, Ru, Co, Ir,
c) introduction de H₂ et de CO,
d) chauffage du mélange réactionnel, l'oléfine étant transformée en aldéhyde.
